# EUROPEAN PATENT APPLICATION

(11) **EP 4 159 167 A1**
(43) Date of publication of application: **05.04.2023**
(21) Application number: 21818360.6
(22) Date of filing: 17.05.2021
(51) Int. Cl.: A61F 2/34

(54) **ACETABULAR IMPLANT**

(30) Priority: 02.06.2020 RU 2020118043
(71) Applicant: Galkin, Anatolii Gerievich, Moskovskaya oblast, g. Korolev, 141079 (RU)
(72) Inventor: Galkin, Anatolii Gerievich, Moskovskaya oblast, g. Korolev, 141079 (RU)
(74) Representative: Studio Torta S.p.A.
(86) International application number: PCT/RU2021/000198
(87) International publication number: WO 2021/246902

(57) **Abstract**

The invention relates to traumatic surgery and orthopedics and is intended for hip acetabular replacement. In a spherical cup of an acetabular implant, in the region of the apex of a hemisphere, a through opening is provided for osteoplasty of the acetabular floor, and the spherical segment of the surface between the edge of the rim of the cup and an opening for a screw for fastening in the ilium is larger than the spherical segments of the surface between said edge and openings for screws for fastening in the pubis and the ischium, thus forming a supporting ledge in the region of the body of the ilium. Said ledge can be extended into the region of an outer spherical surface of the cup which is formed by an angle α in a range of 1° - 30° between diametral planes and which is adjacent to the equatorial plane of the hemisphere. On the portions of the segments of the spherical surface between the edge and the openings for the screws for fastening in the pubis and the ischium, the spherical cup can be additionally cropped to prevent conflict between the soft tissues and implant components. The resulting additional undercut can be cropped in the region of outer spherical surface, which is formed by the angle β between the diametral planes in the range of 1° to 20° and which is adjacent to the equatorial plane of said hemisphere.

## Description

The invention relates to medical technology and is used in traumatic surgery and orthopedics, and intended for the replacement the deformed or destructed acetabulum of a pelvis.

To attach acetabular implants to bone tissue, annular and spiral grooves in variety of shapes [Sivash K.M. Hip Joint Homotransplantation. M.: Medicine, 1967, p70-71, fig. 17 (a, b, v, g)], protrusions, poles [see ibid, p.36, fig.8 (a, b, v, g)], including those having curly members [RU patent 2021785, 1994], as well as bone screws [Moshkovits I.A., Vilensky V.Ya Polymers in Traumatic Surgery and Orthopedics. M.: Medicine, 1978, p139, fig. 62] are used.

All of the above-listed implants designed to enhance the stability of fixation to bone are not sufficiently successful to achieve this. Besides, the acetabular implants with annular grooves provided on their surfaces destruct a bone, when driven into it, resulting in an increased treatment period.

The acetabular implant [RF patent No. 2021786, 1994] in the form of a metal cup including a polymer insert is known. Leaves are arranged on the cup side to provide fixation in the hip joint acetabulum, and inward indentions in the cup are designed for anchoring therein.

However, such construct is unable to anchor securely the endopresthesis.

The acetabular implant [RU patent No. 2131712, 1997] is also known, its feature being that, in the equatorial plane of a hemispherical polymeric cup, an annular recess is formed concentrically with and towards the periphery, away from its socket, giving rise to bores exposed along the same parallel circle of the spherical surface, the bore axes converge in pairs, and attachment elements driven into the bone are in the form of clamps arranged to be moved and fixed in the bores.

The arrangement of bore exit openings along the same parallel circle of the spherical surface of the cup provides a rigid rim formed by clamp extensions, which further stabilizes the implant fixation to bone tissue.

However, the clamps serving as the attachment elements are placed into the bores configured without due regard to the actual load distribution between pelvic bones, which may result in non-uniform stresses and deformities of the rigid rim formed by the clamp extensions 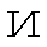 an associated uneven wear of implant-to-bone attachments.

The acetabular component of a hip joint implant is known (RU 2233645 C2, IPC A61F 2/34, date of publication: 10.08.2004). This solution relates to medicine and, in particular, to traumatic surgery, orthopedics, and rheumatology and improves the reliability and durability of insert attachment in the cup, as well as the assemblability control quality for mating parts of the implant component, and thereby extends the service life.

The acetabular component comprises a hemispheric metal cup disposed at the bottom thereof and provided with a double hexagon socket and a groove above the socket, and a polymer insert provided, at its bottom, with a hexagon head circumscribed around the cylindrical part of the insert. The acetabular component also comprises a lock ring. The insert has the conic boss at its top and the hemispherical part for cup contact. At the insert bottom, a groove is cut to at least the depth of ring cross-section. The cup is grooved above the socket. The lock ring is fitted into the insert groove and contacts to the cup groove. Face connection locations are provided with cylindrical channels to at least the depth of cup groove.

The deficiency of the technical solution is the inability to perform osteoplasty of the acetabular floor.

The implant from Mathys Medical Ltd., Switzerland (see Al.A. Nadeev et al "An Efficient Hip Replacement". - M.: BINOM. Knowledge Laboratory, 2004, p.64). This implant is shaped as a spherical cup with concentric corrugations at its exterior. The cup has a hydroxylapatite or titanium-deposition coat, two studs and several cancellous-screw openings differing in diameter. Such a construct of the implant is rather easy-to-use and provides the firm attachment to bone tissue. The above two studs are used for prevention against an overtwisting and primary instability.

However, the separation of functions between strengthening the attachment and stabilizing the orientation with respect to the underlying bone results in a non-uniform distribution of loads on the ilium, the pubis, and the ischium, which raises the risk of the acetabular component instability. In addition, the construct of the implant becomes more cumbersome and elaborate.

An acetabular component of a hip joint implant for the acetabulum defect replacement is known, and it is shaped as a cup provided with a porous overcoat and threaded through openings used to set cancellous screws and having centerlines meeting at a point being a common center of the exterior and interior surface curves of the cup. The cup has a protective rib, internal annular grooves for cement mantle distribution, a threaded opening for an adjustment tool, and the porous coat trabeculate in structure, wherein the cancellous-screw through openings being arranged at the boundaries of spherical layer and provided with internal face recesses intended to receive screw-heads and shaped to deflect the screws through the angle of at least 15°, wherein threaded openings for plugs are made at the faces of recesses for the screw-heads, and the angle between opening centerlines at the boundary of small-diameter spherical layer is by at least 10° more than the angle between opening centerlines at the boundary of large-diameter spherical layer. The openings at the boundary of large-diameter spherical layer have centerlines that are directed at 60° with respect to one another, and the openings at the boundary of small-diameter spherical layer have centerlines that are directed at 72° with respect to one another; in this case, the opening centerlines at the boundary of large-diameter spherical layer are directed at 26° with respect to the cup edge plane, and the opening centerlines at the boundary of small-diameter spherical layer are directed at 58° with respect to the cup edge plane (utility patent RU 195377 U1, IPC A61F 2/34, date of publication: 23.01.2020) .

The deficiencies of this solution are the difficulties in obtaining desired parameters of the cup structure elements and in performing osteoplasty in the acetabular region, despite the adjustment-tool threaded opening that is unusable for osteoplasty because of its very small size.

The acetabular implant (RU patent 2308250 C1, IPC A61F 2/34, date of publication: 20.10.2007) that is to be considered as closest technical solution, namely, the prototype of the proposed invention, comprises a spherical cup provided with through openings for cancellous screws, these screws being provided with a metric screw-thread on the side of their heads and with a cancellous thread having a pitch equal to its metric thread pitch on the rest of their extension up to the screw ends, in this case the thread leads are codirectional, the diameter of the cancellous thread of screws is no more than the diameter of their metric thread, the metric thread having the pitch equal to the cancellous thread pitch is tapped in at least three above-mentioned cancellous-screw through openings, these cancellous-screw openings tapped metrically in the cup are directed so that their axes cross at the angle of 105±15° with respect to one another in a single point coinciding with the common center of the hemispheres forming the cup surface, and the cup is made of metal 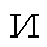 has a porous coat on the exterior.

The openings in the cup are directed so that their axes cross at the angle of 105±15° with respect to one another in the single point being the common center of the hemispheres, which provides the maximum strength, the static and kinematic stability of the construct, and, in combination with the specific threads cut on implant components, the increased stability of fixation of the acetabular implant to bone tissue, the more uniform distribution of implant loads between the pelvic bones, and the improved attachment of the implant to bone tissue in terms of strength, reliability and durability.

The deficiencies of prototype are the difficulties in performing osteoplasty in the regions of the acetabular floor, roof, and back, the inability to provide the reliable fixation of osteoplasty materials, the lack of prevention against collision between the implant components and soft tissues in the lower parts of the acetabulum, and the limitations imposed on the range of implant component sizes due to the equidistance of the cup interior and exterior.

The technical result of the proposed technical solution consists in providing the acetabulum accessibility for bone defect replacement and the improvement in the implant attachment to bone tissue, while concurrently achieving the uniform load distribution between the pelvic bones, the decreased injury to surrounding tissues, the improved ease of osteoplasty performance, the preservation and acquisition of bone mass in an implant zone, as well as the article price being kept at a low level, when widening its range of sizes.

This technical result is achieved due to the fact that, in the acetabular implant comprising a spherical cup provided with through openings for screws for fixation in the ischium, the pubis, and the ilium, a through opening for osteoplasty of the acetabular floor is made in a hemisphere apex region of the spherical cup, and a spherical surface segment between the edge of the rim of said spherical cup and the opening for the screw for fixation in the ilium is larger than respective spherical surface segments between this edge and the openings for the screws for fixation in the pubis and the ischium, respectively, thus forming a supporting ledge in the body region of the ilium.

Additionally, said supporting ledge extends into a region of the outer spherical surface of the cup, which is formed by the angle α between diametral planes in the range of (1° to 30°) and which is adjacent to the equatorial plane of said hemisphere.

Additionally, in the spherical cup, an additional undercut is cropped in spherical segment portions of spherical surface between said edge and the openings for the screws for fixation in the pubis and the ischium, respectively, to prevent any conflict between soft tissues and the implant components.

Additionally, said additional undercut for preventing any conflict between soft tissues and the implant components is cropped in the region of the outer spherical surface, which is formed by the angle β between the diametral planes in the range of (1° to 20°) and which is adjacent to the equatorial plane of said hemisphere.

Additionally, the spherical cup is formed to have a thickness decreasing gradually in the direction from the hemisphere apex towards said edge.

Additionally, the spherical cup is configured to have an insert mounted to create a sliding pair of the joint.

Additionally, the spherical cup is configured to have a plug or an insert element fitted in the through opening for osteoplasty of the acetabular floor.

Additionally, the diameter of the through opening for osteoplasty of the acetabular floor is no less than the double diameter of the openings for attachment screws and no more than 0.75 of the diameter of the hemisphere forming the outer spherical surface of the spherical cup.

Additionally, the outer spherical surface of the cup is prepared to have a porous coat for achieving the biological bone fixation in the acetabulum.

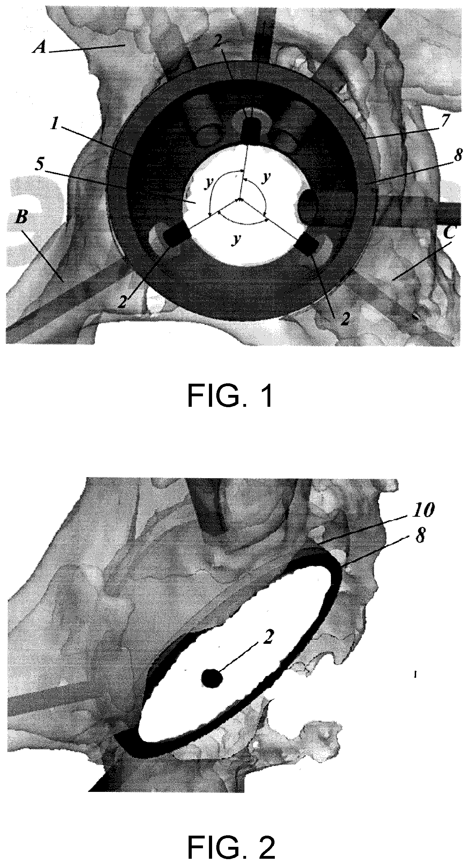

Acetabular implant is shown in Figs. 1 to 6.

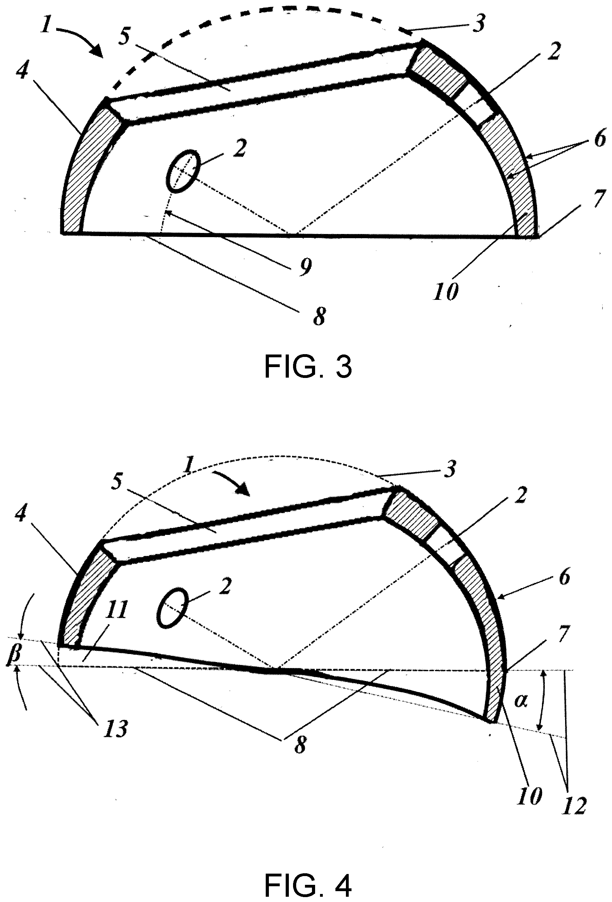

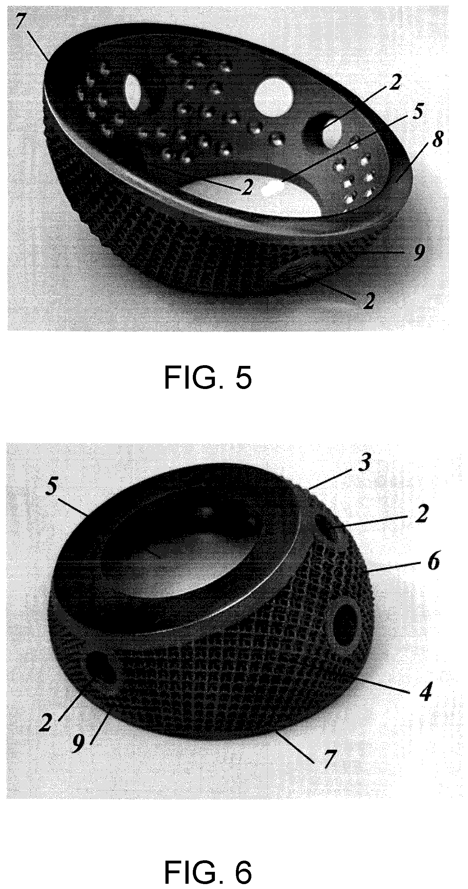

Fig. 1 is a general view of the implant as seen from the equatorial plane of the rim of the implant cup, Fig. 2 is an isometric view of the placed implant cup forming the supporting ledge, as seen from the equatorial plane; Fig. 3 is a schematic cutaway view of the implant cup in the mirror plane of the opening for a screw for fixation in the ilium, with the supporting ledge formed to extend up to the equatorial plane edge; Fig. 4 is a similar cutaway view of the implant cup with the supporting ledge extending into a region of the outer spherical surface of the cup, which is formed by the angle α between diametral planes, and with the additional undercut 11 in the region of the outer spherical surface, which is formed by the angle β between diametral planes; Fig. 5 is an isometric of the implant cup as seen from the equatorial plane; Fig. 6 is a view of the implant cup for osteoplasty of the acetabular floor, as seen from the equatorial plane.

In the drawings, the proposed implementation of both the implant and its spherical cup is shown for the illustration purpose only 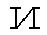 not to be construed as limiting the scope of the invention.

The acetabular implant comprises the spherical cup 1 provided with the through openings 2 for the screws for fixation in the ischium, the pubis, and the ilium, respectively. In this spherical cup 1, the through opening 5 for osteoplasty of the acetabular floor is made in the apex region of the hemisphere 3 so that there is no any conflict between this opening 5 and the screw openings 2, and the spherical segment 6 of the spherical surface between the edge 7 of the equatorial plane 8 of the hemisphere 3 and the opening 2 for the screw for fixation in the ilium is greater than the respective spherical segments 9 of the spherical surface between this edge 7 and the other screw openings 2, thus forming the supporting ledge 10 in the body region of the ilium (Fig. 3). In general, the attachment screws are directed so that their axes cross at the angle Y in the range of 105±15° with respect to one another in a single point coinciding with the common center of the hemisphere 3 forming the outer spherical surface 4 of the cup.

The supporting ledge 10 may be formed in the region of the outer spherical surface 4 of the cup, which is formed by the angle α the between diametral planes 12 (Fig. 4) in the range of (1° to 30°) and which is adjacent to the equatorial plane 8 of said hemisphere 3.

In the spherical cup 1, the additional undercut 11 is cropped in the portions of the spherical segments 9 of the spherical surface between said edge and the openings 2 for the other screws associated with the incisure region of acetabulum to prevent any conflict between soft tissues and the implant components.

This additional undercut 11 for preventing any conflict between soft tissues and the implant components may be cropped in the region of the outer spherical surface, which is formed by the angle β between the diametral planes 13 (φ 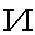 Γ. 4) in the range of (1° to 20°) and which is adjacent to the equatorial plane 8 of said hemisphere 3.

The spherical cup 1 may be formed to have the thickness decreasing gradually in the direction from the apex of the hemisphere 3 towards said edge 7 and beyond, as well as one of common means for mounting the insert (not shown) for creating the sliding pair of the joint.

The spherical cup 1 can be configured to have the plug (not shown) or the insert element fitted in the through opening 5 for osteoplasty of the acetabular floor.

The diameter of the through opening 5 for osteoplasty of the acetabular floor may be no less than the double diameter of the screw openings 2 and no more than 0.75 of the diameter of the hemisphere 3 forming the outer spherical surface 4 of the spherical cup 1.

The outer spherical surface 4 of the cup may be prepared to have the porous coat for achieving the biological bone fixation in the acetabulum.

Such combination of design features provides the acetabulum accessibility via the through opening 5 for bone defect replacement and the improvement in the bone-grafting attachment reliability due to forming the supporting ledge 10 by increasing the portion of the spherical segments 6 of the spherical surface between the edge 7 of the equatorial plane 8 and the openings 2 and by extending this portion into the region between the planes 12, the decreased injury to surrounding tissues due to decreasing the portions of the spherical segments 9 of the spherical surface between said edge and the openings 2 for the other screws, as well as due to cropping the additional undercut 11 in the region between the planes 13, the improved ease of osteoplasty performance, the preservation and acquisition of bone mass in the implant zone, as well as the article price being kept at a low level, when widening its range of sizes by decreasing gradually the wall thickness for the spherical cup 1.

The device is used in the following manner. By one of common methods, the hip joint region is exposed, the joint capsule and scar para-articular tissues are excised and trimmed using one of common methods, and an attachment seat for the above-described construct is formed one size bigger than a size of the implanted acetabular implant by means of a spherical milling cutter.

The proposed on-size spherical cup of the implant is selected and placed in the acetabular region. The cup is rotated so that each of the pelvic bones (A - the ilium, B - the pubis, C - the ischium, see Fig. 1) coincides with at least one attachment opening 2 surrounded by an adjacent spherical-surface portion having a size corresponding to this bone. The cup is secured in the acetabulum by the attachment screws, taking into account the angular stability of attachment screws. The necessary osteoplasty is performed via the through opening 5 at the top of the spherical implant cup, between the body of the ilium, the acetabular backs and the ledge 10.

If a revision arthroplasty and the replacement of the hip joint implant are required, the osteoplasty is performed via the through opening 5 for osteoplasty of the acetabular floor.

Thus, with the proposed solution, the technical result is achieved consisting in providing the accessibility of the acetabular bottom, roof, and backs to perform the replacement of bone defects and improve the reliability of implant attachment to bone tissue, while concurrently distributing uniformly loads between the bones forming the acetabular region, decreasing injury to surrounding tissues, improving the ease of osteoplasty performance, achieving the preservation and acquisition of bone mass in the implant zone, as well as keeping the article price at a low level, when widening its range of sizes.

## Claims

1. An acetabular implant comprising a spherical cup provided with through openings for screws for fixation in the ischium, the pubis, and the ilium, respectively, **characterized in that** a through opening for osteoplasty of the acetabular floor is made in the hemisphere apex region of the spherical cup, and a spherical surface segment between the edge of the rim of said spherical cup and the opening for the screw for fixation in the ilium is larger than respective spherical surface segments between this edge and the openings for the screws for fixation in the pubis and the ischium, respectively, to form a supporting ledge in the body region of the ilium.

2. The implant according to claim 1, **characterized in that** said supporting ledge is extended into the region of the outer spherical surface of the cup, which is formed by the angle α between the diametral planes in the range of (1° to 30°) and which is adjacent to the equatorial plane of said hemisphere.

3. The implant according to claim 1, **characterized in that**, in the spherical cup, an additional undercut is cropped in the spherical segment portions of spherical surface between said edge and the openings for the screws for fixation in the pubis and the ischium, respectively, to prevent a conflict between soft tissues and the implant components.

4. The implant according to claim 3, **characterized in that** said additional undercut for preventing a conflict between soft tissues and the implant components is cropped in the region of the outer spherical surface, which is formed by the angle β between the diametral planes in the range of (1° - 20°) and which is adjacent to the equatorial plane of said hemisphere.

5. The implant according to claim 1, **characterized in that** the spherical cup is formed to have a thickness decreasing gradually in the direction from the hemisphere apex towards said edge.

6. The implant according to claim 1, **characterized in that** the spherical cup is configured to have an insert mounted to create a sliding pair of the joint.

7. The implant according to claim 1, **characterized in that** the spherical cup is configured to have a plug or an insert element fitted in the through opening for osteoplasty of the acetabular floor.

8. The implant according to claim 1, **characterized in that** the diameter of the through opening for osteoplasty of the acetabular floor is no less than the double diameter of the openings for the attachment screws and no more than 0.75 of the diameter of the hemisphere forming the region of the outer spherical surface of the spherical cup.

9. The implant according to claim 1, **characterized in that** the outer spherical surface of the cup is prepared to have a porous coat for achieving the biological bone fixation in the acetabulum.
